# EUROPEAN PATENT APPLICATION

(11) **EP 3 583 969 A1**
(43) Date of publication of application: **25.12.2019**
(21) Application number: 17896783.2
(22) Date of filing: 21.02.2017
(51) Int. Cl.: A61M 11/02, A61M 15/00, B05B 7/02

(54) **INHALER**

(30) Priority: 14.02.2017 JP 2017025010
(71) Applicant: Feather Grass Co., Ltd., Tokyo 103-0013 (JP); Hashiba, Tomohiko, Tokyo 103-0013 (JP)
(72) Inventor: HASHIBA Tomohiko, Tokyo, 1030013 (JP)
(74) Representative: Schön, Christoph
(86) International application number: PCT/JP2017/006369
(87) International publication number: WO 2018/150593

(57) **Abstract**

The present invention relates to an inhaler which is capable of generating super fine particle which has a uniform particle size and has a core-shell structure in which the core comprises a first liquid substance to be sprayed and the shell comprises a second liquid substance to be sprayed. The present invention can spray superfine particles of liquid medicine and the like with a uniform particle size, and can perform coating for sustained release and the like for the superfine particles.

## Description

### TECHNICAL FIELD

The present invention relates to an inhaler or a suction device for sucking superfine particles of a substance to be sprayed such as a medicine and an agrichemical into a person, an animal, or the like.

### BACKGROUND ART

Conventionally, a nebulizer for spraying a liquid medicine in the form of mist has been used for administering it to a patient suffering from respiratory diseases. The nebulizer makes the liquid medicine be in the form of mist by using compressed air or ultrasonic waves for the suction by the patient.

Also, when performing inhalation toxicity tests of environmental pollutants, developments of medicines, and the like, the environmental pollutants, the medicine, and the like in the form of mist are administered to animals for experiments.

It is known that delivery sites of liquid medicine in respiratory system are different depending on a particle size of the liquid medicine to be sprayed. For example, in the case of a human being, the delivery sites are different depending on the particle size of the sprayed liquid medicine as shown below; the throat when the particle size of the liquid medicine is 4.7 to 7 µm, the windpipe when the particle size is 3.3 to 4.7 µm, and the bronchi when the particle size is 1.1 to 3.3 µm.

In conventional nebulizers, the particle size of a liquid medicine to be sprayed could roughly change the particle size of a liquid medicine to be sprayed, depending on, for example, when the delivery site is the nose and when the delivery site is the throat. However, the particle size of a liquid medicine to be sprayed could not be accurately controlled. In addition, since the particle size of a liquid medicine to be sprayed had a certain particle size distribution which was not uniform, the liquid medicine could attach on sites other than the delivery site, and therefore, a large quantity of liquid medicine was required to administer in order to obtain desired medical benefits.

Further, since the particle size of a sprayed environmental pollutants, a liquid medicine, and the like to be used for animals in inhalation toxicity tests of environmental pollutants, developments of medicines, and the like, is not uniform either, errors in the results of the tests or experiments may become large, and accurate test and experimental results may be difficult to obtain.

Thus, JP-A-2003-62074 discloses a suction device which is capable of spraying a liquid medicine and the like with a predetermined uniform particle diameter. The suction device disclosed in JP-A-2003-62074 can make it possible to stably inhale superfine particles with a uniform particle diameter.

### DISCLOSURE OF INVENTION

In case of a liquid medicine in the form of superfine particles which has a very small particle diameter, it is possible to deliver the liquid medicine to the depth of the respiratory system, such as pulmonary alveoli. However, as the pharmaceutical active ingredient in the liquid medicine is rapidly absorbed by a living body and immediately consumed, it may be difficult to maintain the pharmaceutical effects of the liquid medicine.

It may be conceivable to put a coating for sustained release on the superfine particles of a liquid medicine, before the inhalation by a nebulizer. However, as the diameter of the superfine particle of the liquid medicine is very small as compared to a medicine for oral administration such as a tablet, it is difficult to put a coating before being supplied to a nebulizer.

An objective of the present invention is to provide an inhaler which can spray a liquid medicine and the like with a predetermined uniform particle size and can easily perform coating for sustained release or the like on the fine particles of the liquid medicine and the like.

The objective of the present invention can be achieved by an inhaler comprising:
at least one fine particle generating nozzle for generating fine particles comprising a first liquid substance to be sprayed and a second liquid substance to be sprayed by atomizing the first liquid substance and the second liquid substance with gas;
at least one separating vessel for separating the fine particles generated by the fine particle generating nozzle; and
at least one discharging section for discharging superfine particles separated from the fine particles in the separating vessel;
a particle size setting means for setting a particle size of the superfine particles to be discharged from the discharging section;
a particle size detecting means for detecting a particle size of the superfine particles to be discharged from the discharging section; and
a first gas pressure control means for controlling the pressure of a gas to be fed to the fine particle generating nozzle such that the particle size of the superfine particles detected by the particle size detecting means is equal to the particle size of the superfine particles set by the particle size setting means,
wherein
the fine particle generating nozzle comprises
a first injection outlet to inject the first liquid substance to be sprayed into the separating vessel,
a second injection outlet to inject the second liquid substance to be sprayed into the separating vessel, and
a gas injection outlet to inject the gas into the separating vessel,
where
the gas injection outlet is arranged around the first injection outlet and the second injection outlet such that the fine particle generating nozzle is capable of generating a fine particle having a core-shell structure in which the core comprises first liquid substance to be sprayed and the shell comprises the second liquid substance to be sprayed.

The inhaler according to the present invention can spray a liquid substance to be sprayed as superfine particles with a uniform particle size predetermined by a particle size setting means, depending on delivery sites, and can discharge superfine particles of a liquid substance to be sprayed from a discharging section only when being inhaled. Accordingly, it is possible to spray the liquid substance to be sprayed with a particle size depending on a desired delivery site. Also, it is possible to prevent spraying an unnecessary excessive amount of the liquid substance to be sprayed.

The inhaler according to the present invention can easily perform coating the first liquid substance to be sprayed with the second liquid substance to be sprayed. Accordingly, for example, by selecting the first liquid substance to be sprayed from liquid medicines and selecting the second liquid substance to be sprayed from sustained-release materials, the liquid medicine can have sustained releasability and the medicinal efficacy thereof can be maintained.

It is preferable that the fine particle generating nozzle comprises
a nozzle body having the first injection outlet and the second injection outlet, and
a nozzle cover to form a gap around the nozzle body,
where
the gap connects to the gas injection outlet and a source of the gas, and the gas injection outlet is capable of injecting the gas around the first injection outlet and the second injection outlet.

The fine particle generating nozzle can atomize the first liquid substance to be sprayed and the second liquid substance to be sprayed with gas to effectively generate fine particles comprising the first liquid substance to be sprayed and the second liquid substance to be sprayed.

It is preferable that the gas injection outlet be in the shape of a circle. With this feature, it is possible to further enhance the generating efficiency of the fine particles by injecting the gas to the entirety of the first liquid substance to be sprayed and the second liquid substance to be sprayed.

It is preferable that the second injection outlet be arranged around the first injection outlet. With this feature, it is possible to further easily perform coating the first liquid substance to be sprayed with the second liquid substance to be sprayed.

It is preferable that the second injection outlet be in the shape of a circle. It is also preferable that the first injection outlet be arranged in the second injection outlet. By any one of these features, it is possible to further easily make efficiency improvement for the coating of the first liquid substance to be sprayed with the second liquid substance to be sprayed.

It is preferable that the first liquid substance to be sprayed and/or the second liquid substance to be sprayed comprise a liposome. By using a liposome, it is possible to easily perform coating for sustained release. For example, by selecting the first liquid substance to be sprayed from liquid medicines, and by selecting the second liquid substance to be sprayed from liposome-including liquids, it is possible to coat the pharmaceutical active ingredient in the liquid medicine with the liposome for sustained release to maintain the pharmaceutical effects thereof.

It is preferable that the inhaler according to the present invention further comprise a collecting section for collecting the superfine particle which is not inhaled at the discharging section. With this feature, it is possible to prevent adverse effects on environments in case in which the liquid substance to be sprayed has toxicity and the like.

It is preferable that the inhaler according to the present invention further comprise:
a particle amount setting means for setting the amount of superfine particles to be discharged from the discharging section;
a particle amount detecting means for detecting the amount of superfine particles to be discharged from the discharging section; and
a liquid substance amount controlling means for controlling the amount of the first liquid substance to be sprayed and/or the amount of the second liquid substance to be sprayed such that the amount of the superfine particles detected by the particle amount detecting means is equal to the amount of superfine particles set by the particle amount setting means.

According to this embodiment, the particle amount to be discharged from the discharging section is controlled by the liquid substance amount controlling means to the set amount, and therefore, it is possible to appropriately control the particle amount of the liquid substance to be sprayed, depending on the physique, symptoms and the like for human beings, and on the size and the like for animal experiments.

It is preferable that the inhaler according to the present invention further comprise:
a secondary gas feeding means for feeding a secondary gas for elevating the fine particles in the separating vessel; and
a second gas pressure control means for controlling the pressure of the secondary gas to be fed by the secondary gas feeding means such that the particle size of the superfine particles detected by the particle size detecting means is equal to the particle size of the superfine particles set by the particle size setting means.

It is preferable that the separating vessel comprise:
a flow rectifying (e.g., straightening) member for guiding the fine particles generated by the fine particle generating nozzle to a lower part of the separating vessel; and
at least one fine particle sorting plate for controlling floating up of the fine particles guided to the lower part of the separating vessel by the flow rectifying member and for sorting the fine particles.

It is preferable that a plurality of fine particle sorting plates each of which is provided with a plurality of fine particle passing holes through which fine particles pass be disposed inside the separating vessel, and the size of the fine particle passing holes provided at the fine particle sorting plate positioned at the lower side is larger than the size of the fine particle passing holes provided at the fine particle sorting plate positioned at the upper side.

It is preferable that the inhaler according to the present invention further comprise:
an electrical charge supplying means for supplying electrical charges to the superfine particles to be discharged from the discharging section; and
an electrical charge quantity control means for controlling the quantity of electrical charges to be supplied to the superfine particles by the electrical charge supplying means.

According to this embodiment, it is possible to supply positive or negative electrical charge to the superfine particles to be discharged from the discharging section, and to control the electrical charge quantity, and therefore, it is possible to deliver the superfine particles to target sites (with positive or negative electrical charge depending on each site) by controlling the electrical charging thereof.

It is preferable that the vessel wall of the separating vessel have a gas introduction means for introducing gas from outside into the separating vessel.

The present invention also relates to an inhaler comprising:
at least one fine particle generating nozzle for generating fine particles comprising a liquid substance to be sprayed by atomizing the liquid substance to be sprayed with gas;
at least one separating vessel for separating the fine particles generated by the fine particle generating nozzle; and
at least one discharging section for discharging superfine particles separated from the fine particles in the separating vessel;
a particle size setting means for setting a particle size of the superfine particles to be discharged from the discharging section;
a particle size detecting means for detecting a particle size of the superfine particles to be discharged from the discharging section; and
a first gas pressure control means for controlling the pressure of a gas to be fed to the fine particle generating nozzle such that the particle size of the superfine particles detected by the particle size detecting means is equal to the particle size of the superfine particles set by the particle size setting means,
wherein
the vessel wall of the separating vessel has a gas introduction means for introducing gas from outside into the separating vessel.

The inhaler according to this embodiment can perform better the control of size and/or discharging amount of particles.

### EFFECTS OF THE INVENTION

The inhaler according to the present invention can spray a liquid medicine and the like with a predetermined uniform particle size, and can easily perform coating the superfine particles of the liquid medicine and the like for sustained release and the like when the liquid medicine is used.

According to the present invention, it is possible to spray a liquid substance to be sprayed as superfine particles with a uniform particle size predetermined by a particle size setting means, depending on delivery sites, and can discharge the superfine particles of a liquid substance to be sprayed from a discharging section only when being inhaled. Therefore, it is possible to spray the liquid substance to be sprayed with a particle size depending on a desired delivery site. Also, it is possible to prevent spraying an unnecessary excessive amount of the liquid substance to be sprayed.

Also, according to the present invention, it is possible to easily perform coating the first liquid substance to be sprayed with the second liquid substance to be sprayed. Accordingly, for example, by selecting the first liquid substance to be sprayed from liquid medicines and selecting the second liquid substance to be sprayed from sustained-release materials, the liquid medicine can have sustained releasability and the pharmaceutical effects thereof can last. In addition, it is not necessary to coat the first liquid substance to be sprayed with the second liquid substance to be sprayed, before being supplied to the inhaler.

If the inhaler according to the present invention comprises a means for removing electrical charge of fine particles, it is possible to enhance delivery efficiency by making the fine particles have no electrical charge. For example, making fine particles have no electrical charge by removing electrical charge of the fine particles, adhesion of the fine particles to the inner surface of the inhaler by electrostatic action can be reduced, and therefore, discharging efficiency of the fine particles from the discharging section can be enhanced.

If the gas used in the present invention comprise oxygen in a concentration of 50% by volume or more, the inclusion action by a living body for fine particles can be enhanced.

If the first liquid substance to be sprayed and/or the second liquid substance to be sprayed, which is/are used for the present invention, comprise(s) a liposome, it is possible to easily perform coating for sustained release. For example, by selecting the first liquid substance to be sprayed from liquid medicines, and by selecting the second liquid substance to be sprayed from liposome-including liquids, it is possible to coat the pharmaceutical active ingredient in the liquid medicine with the liposome for sustained release to prolong the pharmaceutical effects thereof.

If the inhaler according to the present invention further comprises a collecting section, it is possible to collect the superfine particle of the liquid substance to be sprayed which is not inhaled. Thus, it is possible to prevent adverse effects on environments if the liquid substance to be sprayed has toxicity and the like.

If the inhaler according to the present invention further comprise a liquid substance amount controlling means, the amount of the superfine particles to be discharged from the discharging section can be controlled by the liquid substance amount controlling means to be a predetermined amount, and therefore, it is possible to appropriately control the particle amount of the liquid substance to be sprayed, depending on the physique, symptoms and the like of a person if a human being inhales, and on the size and the like of an animal if an animal inhales in animal experiments.

If the inhaler according to the present invention can provide the superfine particles to be discharged from the discharging section with electrical charge, and can control the amount of the electrical charge, it is possible to deliver the superfine particles to target sites by controlling the type of the electrical charge (positive or negative) and amount of electrical charge.

If the vessel wall of the separating vessel in the inhaler according to the present invention have a gas introduction means for introducing gas from outside into the separating vessel, it is possible to control better the particle size and/or the discharging amount.

### BRIEF DESCRIPTIONS OF THE DRAWINGS

FIG. 1 shows a schematic configuration view of an embodiment of the inhaler according to the present invention.
FIG. 2 shows an enlarged cross-sectional view of the fine particle generating nozzle in an embodiment of the inhaler according to the present invention.
FIG. 3 shows an enlarged front view of a fine particle sorting plate in an embodiment of the inhaler according to the present invention.
FIG. 4 shows an enlarged front view of another fine particle sorting plate in an embodiment of the inhaler according to the present invention.
FIG. 5 shows an enlarged front view of the other fine particle sorting plate in an embodiment of the inhaler according to the present invention.
FIG. 6 shows a system diagram of an embodiment of the inhaler according to the present invention.

### BEST MODE FOR CARRYING OUT THE INVENTION

Hereinafter, an embodiment of the inhaler according to the present invention is described with reference to the figures.

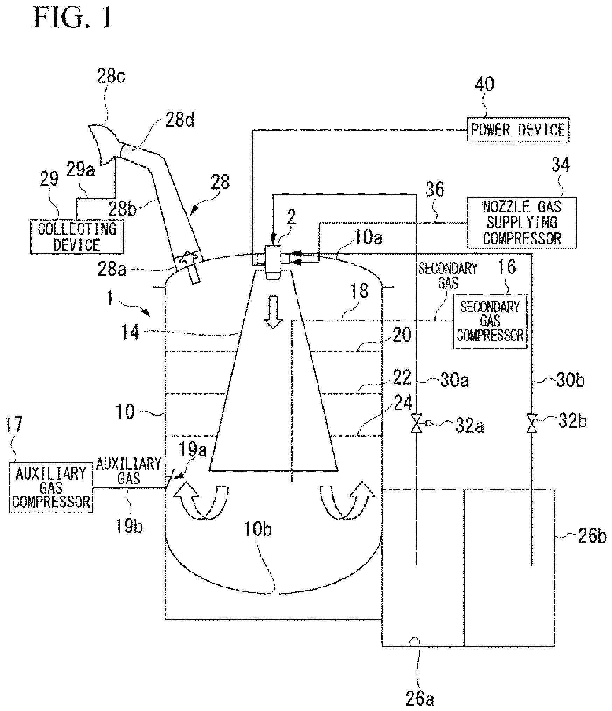

As shown in Fig. 1, an inhaler 1 according to an embodiment of the present invention comprises a cylindrical fine particle separating vessel 10 whose lower end is closed and whose upper end is provided with a lid portion 10a. The lid portion 10a of the fine particle separating vessel 10 is provided with a three-fluid nozzle (fine particle generating nozzle) 2 for atomizing first and second liquid substances to be sprayed by gas such as air.

First, the three-fluid nozzle 2 is explained.

The three-fluid nozzle 2 is to inject gas from a gas injection outlet toward the outer peripheral portion of the first and second liquid substances to be sprayed which are injected from injection outlets provided at a tip end of the nozzle, and to generate a fine particle having a core-shell structure in which the core comprises the first liquid substance to be sprayed and the shell comprises the second liquid substance to be sprayed by atomizing the first and second liquid substances by the gas.

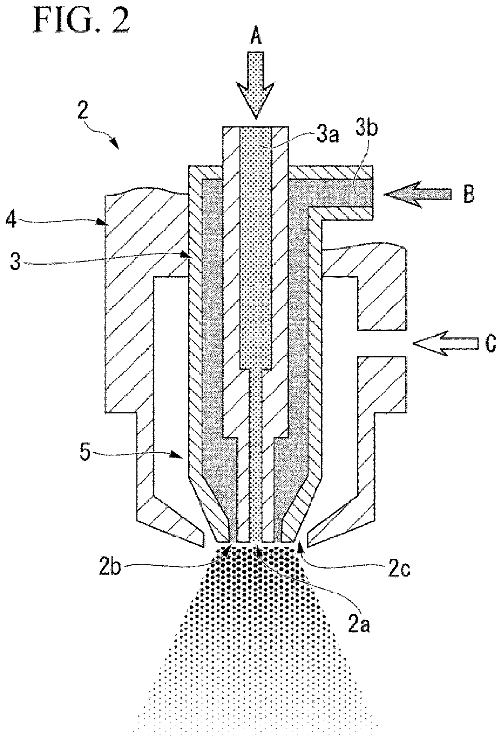

As shown in Fig. 2, the three-fluid nozzle 2 comprises a first injection outlet 2a which injects, into the fine particle separating vessel 10, the first liquid substance (A) to be sprayed, a second injection outlet 2b which injects, into the fine particle separating vessel 10, the second liquid substance (B) to be sprayed, and a gas injection outlet 2c which injects gas (C) into the fine particle separating vessel 10. The gas injection outlet 2c is arranged around the first injection outlet 2a and the second injection outlet 2b.

Specifically, in the embodiment shown in Fig. 2, the three-fluid nozzle 2 comprises a nozzle body 3 having the first injection outlet 2a and the second injection outlet 2b. In the nozzle body 3, a first conduit 3a to supply the first liquid substance (A) to be sprayed from a supply source which is not shown in the figure to the first injection outlet 2a, and a second conduit 3b to supply the second liquid substance (B) to be sprayed from another supply source which is not shown in the figure to the second injection outlet 2b, are formed.

Around the nozzle body 3, a nozzle cover 4 to form a gap 5 is arranged. The gap 5 connects the gas injection outlet 2c and a gas supply source which is not shown in the figure. The gas injection outlet 2c is capable of injecting the gas (C) around the first injection outlet 2a and the second injection outlet 2b.

In the embodiment shown in Fig. 2, the gas injection outlet 2c is in the shape of a circle, and surrounds the first injection outlet 2a and the second injection outlet 2b. Therefore, it is possible to enhance the generating efficiency of the fine particles by injecting gas (C) from the gas injection outlet 2c to the whole of the first liquid substance (A) to be sprayed which is injected from the first injection outlet 2a and the second liquid substance (B) to be sprayed which is injected from the second injection outlet 2b.

Further, in the embodiment shown in Fig. 2, the second injection outlet 2b is arranged around the first injection outlet 2a. Therefore, it is possible to easily coat the first liquid substance (A) to be sprayed with the second liquid substance (B) to be sprayed. Furthermore, in the embodiment shown in Fig. 2, the second injection outlet 2b is in the shape of a circle. The second injection outlet 2b surround the first injection outlet 2a. Specifically, the first injection outlet 2a is arranged in the second injection outlet 2b. Therefore, it is possible to make it more efficient to coat the first liquid substance (A) to be sprayed with the second liquid substance (B) to be sprayed.

When generating fine particles from the three-fluid nozzle 2 in the embodiment shown in Fig. 2, the first liquid substance (A) to be sprayed, the second liquid substance (B) to be sprayed and gas (C) are injected from the first injection outlet 2a, the second injection outlet 2b and the gas injection outlet 2c, respectively. Thus, the first liquid substance (A) to be sprayed is coated with the second liquid substance (B) to be sprayed, and atomized by the gas (C). As a result, fine particles having a core-shell structure in which the core comprises the first liquid substance (A) to be sprayed and the shell comprises the second liquid substance (B) to be sprayed are injected from the three-fluid nozzle 2.

As explained above, the three-fluid nozzle 2 can efficiently generate fine particles comprising the first and second liquid substances to be sprayed by atomizing the first and second liquid substances to be sprayed with gas. The fine particles can have a core-shell structure in which the core comprises the first liquid substance (A) to be sprayed and the shell comprises the second liquid substance (B) to be sprayed.

In summary, it is possible by the three-fluid nozzle 2 to easily coat the first liquid substance to be sprayed with the second liquid substance to be sprayed, and to generate fine particles which can maintain the coating form.

In the present invention, it is not necessary to coat beforehand the first liquid substance to be sprayed with the second liquid substance to be sprayed before being supplied to the inhaler, because the first and second liquid substances to be sprayed are mixed, just before spraying, to coat the first liquid substance to be sprayed with the second liquid substance to be sprayed. Also, as the coating is performed by the injection from the fine particle generating nozzle, the coating of the first liquid substance to be sprayed with the second liquid substance to be sprayed can be easily performed.

The first and second liquid substances to be sprayed are in the form of a liquid at an ambient temperature (25°C). It is preferable that they are in the form of a liquid at a lower temperature (e.g., 0°C).

It is preferable that the first and second liquid substances to be sprayed have fluidity at an ambient temperature (25°C). The "fluidity" at 25°C means that, first the liquid surface of 1 kg of a liquid substance in a certain vessel is made horizontal, second the vessel is inclined, and then the liquid surface thereof can be horizontal again within 12 hours, preferably 6 hours, more preferably 1 hour, and even more preferably 30 minutes after inclining the vessel. The "horizontal" here means the flat plane which cross at right angle with the action direction of gravity.

The viscosity of the first or second liquid substance to be sprayed is not limited. Thus, the present invention can handle, as the first or second liquid substance to be sprayed, a variety of liquids from a low viscous liquid to a high viscous liquid. For example, as the first or second liquid substance to be sprayed, a liquid with a viscosity of 1^{∗}10⁻⁴ to 100 Pa.s, preferably from 5^{∗}10⁻⁴ to 50 Pa.s and more preferably from 1^{∗}10⁻³ to 10 Pa.s, at 25°C, can be used.

The first and/or the second liquid substance(s) to be sprayed may be in the form of a solution in which any substance that is in the form of a solid at an ambient temperature (25°C) is solubilized in an appropriate solvent(s). As the solvent, a volatile or non-volatile one may be used. A volatile solvent such as water is preferable. For example, as the first liquid substance to be sprayed, a liquid medicine in which a powder of an active pharmaceutical ingredient that is in the form of a solid at an ambient temperature is solubilized in a volatile solvent may be used. As the second liquid substance to be sprayed, a coating liquid in which a coating substance (e.g., sustained-release material) that is in the form of a solid at an ambient temperature is solubilized in a volatile solvent may be used. Thus, composite fine particles in which fine particles of the active pharmaceutical ingredient are coated with the coating substance can be generated.

The sustained-release material is not limited. For example, synthetic polymers such as polyvinylalcohol (PVA), polyethyleneglycol (PEG), and acrylic polymers; natural polymers such as guar gum, acacia gum, tragacanth gum, and xanthan gum; cellulose polymer such as HPMC, hydroxypropylcellulose (HPC), hydroxyethylcellulose (HEC), methylcellulose (MC), ethylcellulose (EC), and carboxymehtylcellulose (CMC); sugar alcohols such as xylitol, sorbitol and maltitol; proteins such as gelatin; and other materials, may be used.

According to one embodiment of the present invention, by using a liquid medicine as the first liquid substance to be sprayed, and by using a sustained release material as the second liquid substance to be sprayed, it is possible to make the liquid substance to have sustained-releasability, thereby lasting or maintaining the pharmaceutical effects thereof.

It is preferable that the first and/or second liquid substance(s) to be sprayed to include a liposome. A liposome can function as a sustained-release material, and therefore, it is possible to easily make a sustained-release coating by using a liposome. For example, by using a liquid medicine as the first liquid substance to be sprayed, and by using a liposome-including liquid as the second liquid substance to be sprayed, it is possible to make the liquid substance to have sustained-releasability by coating the active pharmaceutical ingredient in the liquid medicine with the liposome, thereby lasting or maintaining the pharmaceutical effects thereof.

It was necessary in conventional preparation technology to use a hydrophobic solvent, which needed care handling, for the preparation of a liposome complex. On the other hand, finally, the hydrophobic solvent needed to be removed. Thus, conventional preparations required additional steps, and efficient preparation was difficult. However, the present invention can use a hydrophilic solvent such as water, which is easy to handle, and therefore, it is possible to realize efficient preparation of a liposome-coated fine particle, by simplifying preparation steps.

If the fine particles generated by the fine particle generating nozzle in the inhaler according to the present invention includes an active pharmaceutical ingredient, the active pharmaceutical ingredient does not denature, and therefore, the present invention does not impair the pharmaceutical effects of the active pharmaceutical ingredient.

Accordingly, as the fine particle used in the present invention, a vaccine may be used. For example, by using a liquid medicine including a certain antigen as the first liquid substance to be sprayed, and by using a solvent for the medicine as the second liquid substance to be sprayed, it is possible to make vaccine fine particles. In this case, as the inhaler according to the present invention can avoid physical actions such as heat and pulverizing power, against the active pharmaceutical ingredient (antigen), the active pharmaceutical ingredient does not denature, and the titer or potency of the vaccine does not change. The fine particles used in the present invention may also include a variety of biological agents such as IPS cells.

The gas to be used for the present invention is not limited. Thus, as the gas, for example, nitrogen, oxygen, or a mixture of various gases, such as air, which includes nitrogen, oxygen and the like, may be used. By controlling the type of the gas, it is possible to control the amount of gas dissolved in the first liquid substance to be sprayed and the second liquid substance to be sprayed. It is preferable that the oxygen concentration of the gas be 50% by volume, more preferably 80% by volume, and even more preferably the gas is pure oxygen. With this, it is possible to enhance the inclusion action by a living body for the fine particles. Also, it is possible to make the amount of oxygen dissolved in the first liquid substance to be sprayed and the second liquid substance to be sprayed be a saturated amount, thereby the inclusion action by a living body for the fine particles can also be enhanced.

In the embodiment shown in Fig. 2, a three-fluid nozzle 2 which has a relatively simple structure is used. Thus, a relatively viscous or thick liquid can be used as the first and/or second liquid substance to be sprayed. In addition, it is difficult to cause clogging. Also, it can have excellent wear resistance. The materials for the elements, such as the nozzle body 3 and the nozzle cover 4, of the three-fluid nozzle 3 are not limited. For example, stainless steel such as SUS 304, and anti-wear plastic such as Teflon® may be mentioned.

The shape or area of at least one outlet among the first injection outlet 2a, the second injection outlet 2b and the gas injection outlet 2c may be changeable. The supply amount and/or the supply speed of at least one of the first liquid substance to be sprayed, the second liquid substance to be sprayed and the gas can be easily controlled. For example, the relative position between the nozzle body 3 and the nozzle cover 4 may be changeable to increase or decrease the outlet area of the gas injection outlet 2c, in order to control the injection amount and/or the injection speed of the gas for controlling the size of the fine particles or for controlling the coating degree of the first liquid substance to be sprayed with the second liquid substance to be sprayed.

Next, the fine particle separating vessel 10 is explained.

As shown in Fig. 1, there is provided inside the fine particle separating vessel 10 a flow rectifying cone (flow rectifying member) 14 for flow-straightening and guiding the fine particles generated by the three-fluid nozzle 2 to a lower part of the fine particle separating vessel 10. Here, the tip end of the three-fluid nozzle 2 is disposed at an upper opening of the flow rectifying cone 14.

Further, a secondary gas feeding pipe 18 extending from a secondary gas compressor 16 is disposed inside the fine particle separating vessel 10, and a secondary gas such as air from the secondary gas compressor 16 is fed to the vicinity of a lower opening of the flow rectifying cone 14. Further, there are provided inside the fine particle separating vessel 10 fine particle sorting plates 20, 22, 24 for controlling floating up of the fine particles guided to the lower part of the fine particle separating vessel 10 by the flow rectifying cone 14, and for sorting or separating the fine particles. The number of the fine particle sorting plates is not limited, and can be appropriately changed.

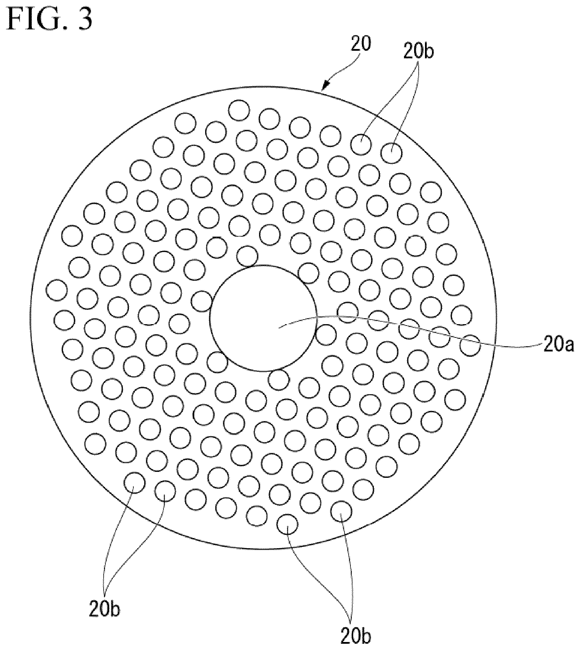

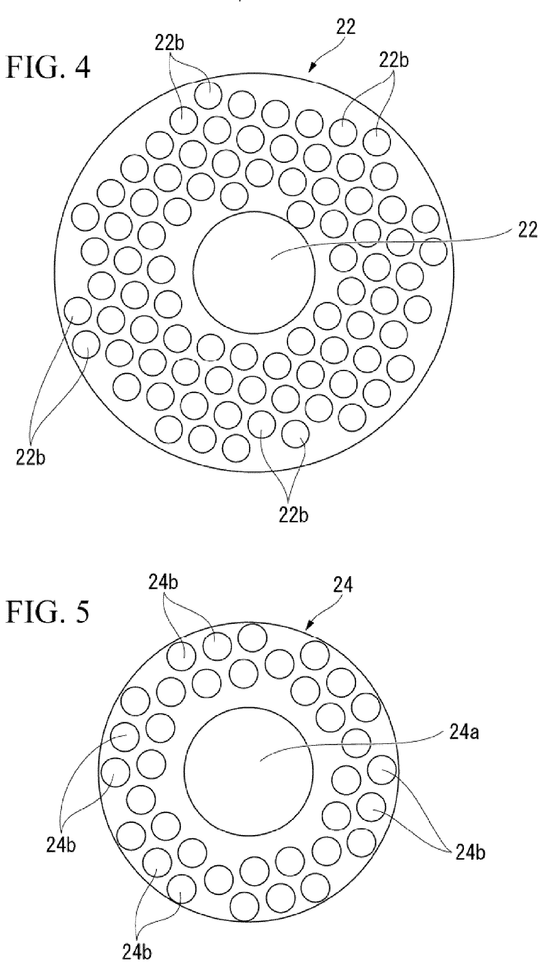

As shown in Fig. 3, the fine particle sorting plate 20 is a plate-shape member of a circular plate shape where an opening 20a through which the flow rectifying cone 14 penetrates is provided at the center thereof, and is provided with a large number of fine particle passing holes 20b. Further, as shown in Fig. 4, the fine particle sorting plate 22 is a plate-shape member of a circular plate shape where an opening 22a through which the flow rectifying cone 14 penetrates is provided at the center thereof, and is provided with a large number of fine particle passing holes 22b. The fine particle passing holes 22b are made to be larger than the fine particle passing holes 20b of the fine particle sorting plate 20. Furthermore, as shown in Fig. 5, the fine particle sorting plate 24 is a plate-shape member of a circular plate shape where an opening 24a through which the flow rectifying cone 14 penetrates is provided at the center thereof, and is provided with a large number of fine particle passing holes 24b. The fine particle passing holes 24b are made to be larger than the fine particle passing holes 22b of the fine particle sorting plate 22.

A discharge port 10b is provided at the bottom of the fine particle separating vessel 10. The fine particles retained at the bottom of the fine particle separating vessel 10 is discharged from the discharge port 10b.

Further, a discharging section 28 for discharging superfine particles separated from the fine particles inside the fine particle separating vessel 10 is provided at the lid portion 10a of the fine particle separating vessel 10. Here, the discharging section 28 is configured with a spray port attaching portion 28a attached at the lid portion 10a of the fine particle separating vessel 10, a spray guiding conduit 28b connected to the spray port attaching portion 28a, and a suction port 28c provided at a tip end of the spray guiding conduit 28b. There is provided a valve 28d for opening only during suction between the spray guiding conduit 28b and the suction port 28c. Further, the suction port 28c is provided with a collecting device 29 for collecting the superfine particles via a superfine particle collecting pipe 29a.

In the embodiment shown in Fig. 1, there is provided only one discharging section 28. However, there is no limitation for the numbers of the discharging section 28 to be provided. For example, the fine particle separating vessel 10 may have a plurality of discharging sections, thereby superfine particles can be delivered to a number of patients at the same time.

In the embodiment shown in Fig. 1, an auxiliary gas nozzle 19a is provided at the vessel wall of the fine particle separating vessel 10, and is connected to an auxiliary gas supplying pipe 19b extended from an auxiliary gas compressor 17. The injection outlet of the auxiliary gas nozzle 19a is directed to the discharging section 28. The auxiliary gas supplied from the auxiliary gas compressor 17 is discharged, in the fine particle separating vessel 10, upwardly from the auxiliary gas nozzle 19a. In the embodiment shown in the figure, the auxiliary gas compressor 18, the auxiliary gas supplying pipe 19b, and the auxiliary gas nozzle 19a constitutes a gas introduction means for introducing gas from outside into the separating vessel.

In the embodiment shown in Fig. 1, there are provided storing vessels 26a, 26b, which store the first liquid substance to be sprayed and the second liquid substance to be sprayed, respectively, along with the fine particle separating vessel 10. Further, there are provided supplying pipes 30a, 30b, for supplying the first liquid substance to be sprayed and the second liquid substance to be sprayed, respectively, to the three-fluid nozzle 2, between the storing vessels 26a, 26b, and the three-fluid nozzle 2. The supplying pipes 30a, 30b are provided with solenoid valves 32a, 32b for adjusting the quantity of each of the liquid substances to be supplied to the three-fluid nozzle 2. Further, there is provided a gas feeding pipe 36 for feeding a nozzle gas to the three-fluid nozzle 2 between the nozzle gas supplying compressor 34 and the three-fluid nozzle 2.

Further, in the embodiment shown in Fig. 1, a power device 40 for supplying electrical charges to the superfine particles to be discharged from the suction port 28c of the discharging section 28 is connected to the three-fluid nozzle 2. A desired DC voltage can be supplied to the three-fluid nozzle 2c by this power device 40 so that the superfine particles discharged from the suction port 28c are charged to be + or -. If the superfine particles are charged in this manner, it is possible to improve attachment efficiency of the superfine particles to an application or delivery site.

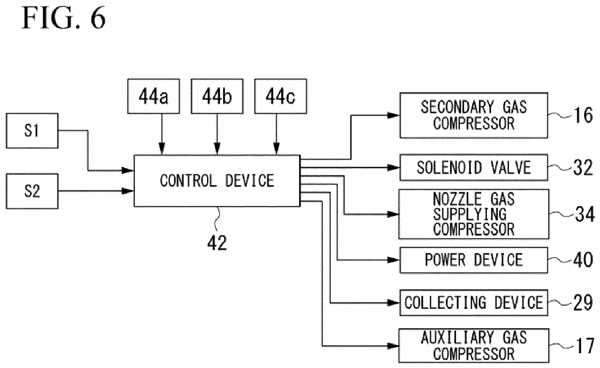

A pressure of the secondary gas to be fed from the above secondary gas compressor 16 into the fine particle separating vessel 10, a pressure to be fed from the auxiliary gas compressor 18 to the fine particle separating vessel 10, a pressure of the nozzle gas to be fed to the three-fluid nozzle 2 by the nozzle gas supplying compressor 34, and the quantity of the liquid substance(s) to be supplied to the three-fluid nozzle 2 via the solenoid valves 32a, 32b can be controlled by a control device 42 (refer to Fig. 6). Further, the type (+ or -) of electrical charges and the quantity of electrical charges (the quantity of electrostatic charges) to be supplied to the three-fluid nozzle 2 by the power device 40 can be controlled by the control device 42. In other words, the control device 42 can function as a first gas pressure control means for controlling a pressure of the nozzle gas to be fed to the three-fluid nozzle 2, a second gas pressure control means for controlling a pressure of the secondary gas to be fed into the fine particle separating vessel 10, an auxiliary gas pressure control means for controlling a pressure of the auxiliary gas to be fed into the fine particle separating vessel 10, a liquid substance quantity control means for controlling the quantity of the liquid substance to be supplied to the three-fluid nozzle 2, and an electrical charge control means for controlling the type and quantity of electrical charges to be supplied to the suction port 28c of the discharging section 28.

Further, a particle size detecting section S1 for detecting a distribution of particle sizes of the superfine particles to be discharged from the suction port 28c of the discharging section 28, and a particle quantity detecting section S2 for detecting the particle quantity of the superfine particles are connected to the control device 42, and detection values from the particle size detecting section S1 and the particle quantity detecting section S2 are input therein. Furthermore, the control device 42 are connected to a particle size setting section 44a for setting a particle size of the superfine particles to be discharged from the suction port 28c of the discharging section 28, a particle quantity setting section 44b for setting the particle quantity of the superfine particles, and an electrical charge setting section 44c for setting the type and quantity of electrostatic charges (quantity of electrical charges to be supplied) of the superfine particles.

Next, generation of superfine particles by this inhaler 1 will be described. In the following description, a description will be given by way of an example where the first and second liquid medicines to be sprayed are supplied to the three-fluid nozzle 2, and air, as a nozzle gas, a secondary gas and an auxiliary gas, is supplied so that the superfine particles of the first and second liquid substances to be sprayed are generated to be sucked by a person.

First, a particle size of the superfine particles of the first and second liquid substances to be discharged from the suction port 28c of the discharging section 28 is set by the particle size setting section 44a, and the particle quantity of the superfine particles to be discharged from the suction port 28c of the discharging section 28 is set by the particle quantity setting section 44b. In other words, an appropriate particle size is set according to a site to be medicated, and the particle quantity is set according to a body type, a symptom, and the like of a person to be medicated.

After the setting of the particle size of the superfine particles and the setting of the particle quantity of the superfine particles are terminated, the superfine particles of the first and second liquid substances to be sprayed are generated. In other words, in this inhaler 1, when air (nozzle gas) is fed from the nozzle gas supplying compressor 34 to the three-fluid nozzle 2 via the gas feeding pipe 36, this air is injected from the gas injection outlet 2c at the tip end of the three-fluid nozzle 2 (cf. Fig. 2), and the first and second liquid substances to be sprayed inside the storing vessels 26a, 26b are sucked up by this injection force and supplied to the three-fluid nozzle 2 via the supplying pipes 30a, 30b.

In the three-fluid nozzle 2, as mentioned above, the first liquid substance to be sprayed and the second liquid substance to be sprayed, which will be injected from the first injection outlet 2a and the second injection outlet 2b (cf. Fig. 2), are atomized by the air injected from the gas injection outlet 2c (cf. Fig. 2) so that the fine particles of the first and second liquid substances are injected. The fine particles injected from this three-fluid nozzle 2 are guided through the flow rectifying cone 14 to the lower part of the fine particle separating vessel 10. On the other hand, the air (secondary gas) from the secondary gas compressor 16 is fed to the vicinity of the lower opening of the flow rectifying cone 14 via the secondary gas feeding pipe 18. In addition, the air (auxiliary gas) from the auxiliary gas compressor 17 is fed into the fine particle separating vessel 10 via the auxiliary gas supplying pipe 19b, and discharged from the auxiliary gas nozzle 19a toward the discharging section 28.

While the fine particles guided to the lower part of the fine particle separating vessel 10 are trying to float up or elevate due to an upward flow of the air (nozzle air) fed from the three-fluid nozzle 2, the secondary air and the auxiliary air, floating us or elevating is restricted or controlled by the fine particle sorting plates 20, 22, and 24. Thus, they gradually or slowly float up or elevate inside the fine particle separating vessel 10 through the fine particle passing holes 20b, 22b, and 24b provided at the fine particle sorting plates 20, 22, and 24, respectively. In other words, first, the fine particles passed through the fine particle sorting plate 24 are restricted from floating up or elevating by the fine particle sorting plate 22, and fine particles having a predetermined particle size are retained between the fine particle sorting plate 24 and the fine particle sorting plate 22. Here, fine particles having a large particle size drop on the bottom of the fine particle separating vessel 10 due to the force of gravity.

Then, the fine particles passed through the fine particle sorting plate 22 are restricted from floating up or elevating by the fine particle sorting plate 20, and the fine particles having a predetermined particle size are retained between the fine particle sorting plate 22 and the fine particle sorting plate 20. Here, fine particles having a large particle size drop on the bottom of the fine particle separating vessel 10 due to the force of gravity. The particle size of the fine particles retained between the fine particle sorting plate 22 and the fine particle sorting plate 20 is made smaller than the particle size of the fine particles retained between the fine particle sorting plate 24 and the fine particle sorting plate 22.

As the fine particles float up or elevate inside the fine particle separating vessel 10 in this manner, the fine particles having a large particle size drop on the bottom of the fine particle separating vessel 10, and only the superfine particles having a uniform particle size are discharged from the discharging section 28 of the fine particle separating vessel 10. The first and second liquid substances retained at the bottom of the fine particle separating vessel 10 are discharged from the discharge port 10b.

A distribution of the particle size of the superfine particles discharged from the suction port 28c of the discharging section 28 is detected by the particle size detecting section S1 and the particle quantity of the superfine particles is detected by the particle quantity detecting section S2. The detection values detected by the particle size detecting section S1 and the particle quantity detecting section S2 are input into the control device 42. In the control device 42, control signals are output to the secondary gas compressor 16, the auxiliary gas compressor 17, the solenoid valves 32a, 32b, and the nozzle gas supplying compressor 34, in order to control the pressure of the air (nozzle gas) to be fed to the three-fluid nozzle 2 and the quantity of the first and second liquid substances to be supplied to the three-fluid nozzle 2 and the pressure of the secondary gas and the auxiliary gas to be fed to the fine particle separating vessel 10, such that the particle size of the superfine particles to be discharged from the suction port 28c of the discharging section 28 is equal to a value set by the particle size setting section 44a, and the particle quantity of the superfine particles is equal to a value set by the particle quantity setting section 44b.

In other words, in the control device 42, when the particle size of the superfine particles detected by the particle size detecting section S1 is larger than that set by the particle size setting section 44a, the nozzle gas supplying compressor 34 is controlled so that the pressure of the air (nozzle gas) to be fed to the three-fluid nozzle 2 is increased. Also, the secondary gas compressor 16 can be controlled to increase the pressure of the air (secondary gas) to be fed into the fine particle separating vessel 10, and/or the auxiliary gas compressor 17 can be controlled to increase the pressure of the air (auxiliary gas) to be fed into the fine particle separating vessel 10. Thereby, the particle size of the superfine particles to be discharged from the suction port 28c can be made smaller.

On the other hand, when the particle size of the superfine particles detected by the particle size detecting section S1 is smaller than that set by the particle size setting section 44a, the nozzle gas supplying compressor 34 is controlled to reduce the pressure of the air (nozzle gas) to be fed to the three-fluid nozzle 2. Also, the secondary gas compressor 16 can be controlled to reduce the pressure of the air (secondary gas) to be fed into the fine particle separating vessel 10, and/or the auxiliary gas compressor 17 can be controlled to reduce the pressure of the air (auxiliary gas) to be fed into the fine particle separating vessel 10. Thereby, the particle size of the superfine particles to be discharged from the suction port 28c can be made smaller.

Although either the pressure of the air (nozzle gas) to be fed to the three-fluid nozzle 2 or the pressure of the air (secondary gas and/or the auxiliary gas) to be fed into the fine particle separating vessel 10 can be controlled so that the particle size of the superfine particles to be discharged from the suction port 28c can be controlled, both the pressure of the air (nozzle gas) to be fed to the three-fluid nozzle 2 and the pressure of the air (secondary and/or auxiliary gas) to be fed into the fine particle separating vessel 10 can be controlled so that the particle size of the superfine particles can be controlled more accurately.

Further, when the quantity of the fine particles detected by the particle quantity detecting section S2 is smaller than the quantity set by the particle quantity setting section 44b, control signals are output to the solenoid valves 32a, 32b, to increase the quantity of the first and/or second liquid substance(s) to be supplied to the three-fluid nozzle 2. Thereby, the particle quantity of the superfine particles to be discharged from the suction port 28c can be increased. On the other hand, when the quantity of the fine particles detected by the particle quantity detecting section S2 is larger than the quantity set by the particle quantity setting section 44b, control signals are output to the solenoid valves 32a, 32b to reduce the quantity of the first and/or second liquid substance(s) to be supplied to the three-fluid nozzle 2. Thereby, the particle quantity of the superfine particles to be discharged from the suction port 28c can be reduced.

In this inhaler 1, the above control may be repeated until the particle size of the superfine particles detected by the particle size detecting section S1 is equal to the particle size set by the particle size setting section 44a and the particle quantity of the liquid medicine detected by the particle quantity detecting section S2 is equal to the particle quantity set by the particle quantity setting section 44b, and then suction can be started.

In this inhaler 1, the valve 28 can be opened only during suction so that the superfine particles can be delivered/sucked from the suction port 28c. Further, the superfine particles which have been delivered to the suction port 28c but have not been sucked therefrom can be collected by the collecting device 29.

According to the inhaler 1 of this embodiment, the first and second liquid substances can be sprayed as superfine particles having a uniform particle size set by the particle size setting means, and the superfine particles can be discharged only during suction. Since the superfine particles can be sprayed at the particle size according to a desired application or delivery site, large medical benefits can be obtained by a small quantity of the first and second liquid substances. Further, since it is possible to prevent an excessive amount of the first and second liquid substance from being sprayed, the consumption of the first and second liquid substances can be reduced.

Further, the first and second liquid substances are sprayed as superfine particles having a uniform particle size set by the particle size setting means, while the superfine particles which have not been sucked up can be collected by the collecting section. Therefore, it is possible to prevent the substances from affecting the environment even when the first and second liquid substances are, for example, toxic.

Furthermore, since the particle quantity of the superfine particles to be discharged from the suction port 28c can be controlled to be the set quantity, the particle quantity of the first and/or second liquid substance(s) can be accurately controlled according to a body type and a symptom of a person if the person inhales.

Originally, the superfine particles which can be generated by the present invention are not electrically charged. However, in the inhaler 1 according to the above embodiment, the superfine particles to be discharged from the suction port 28c of the discharging section 28 can be charged as + or - by the electrical charge setting section 44c. In other words, when the type of charge (+ or -) and the electrostatic charge quantity are set by the electrical charge setting section 44c, the control device 42 outputs a control signal to the power device 40 on the basis of the set value, and the type and quantity of electrical charges to be supplied to the three-fluid nozzle 2 is controlled by the power device 40. Therefore, the type and electrical charge quantity of superfine particles can be controlled so that the superfine particles can be securely attached on the application or delivery site. Also, as the target to be electrically charged by the present invention is fine particles, the electrical power necessary for charging may be small such as 1kw or less.

On the other hand, superfine particles may not be electrically charged. Thus, in the inhaler according to the embodiment shown in Fig. 1, it may not be necessary to connect the power device 40 to the three-fluid nozzle 2 to electrically charge superfine particles generated from the three-fluid nozzle2. Also, for example, the discharging section 28 may have an electrical charge removing means for removing electrical charge on superfine particles. If the inhaler according to the present invention has an electrical charge removing means for fine particles, the fine particles can have no electrical charge, and therefore, the delivery efficiency of the fine particles may be enhanced. For example, by making fine particles have no electrical charge by removing electrical charges from the fine particles, the adhesion of the fine particles onto the inner surface of the inhaler can be reduced, and therefore, discharging efficiency of the fine particles from the discharging section can be enhanced.

Further, although a description has been given by way of an example where the superfine particles of the first and second liquid substances are generated to be sucked by a person in the above embodiment, liquid medicines, environmental pollutants, and the like may be treated as a substance to be sprayed, and the superfine particles of this substance to be sprayed can be sucked by animals so that animal experiments can be performed. In this case, since a set uniform superfine particles can be used for the experiments, accurate experiment results can be obtained. Also, plants may suck fine particles via stomata from outside, and therefore, the present invention may be useful for plants as well.

Furthermore, in the above embodiment, although when air is fed to the three-fluid nozzle 2, this air is injected from the gas injection outlet at the tip end of the three-fluid nozzle 2, and the first and second liquid substances to be sprayed in the storing vessels 26a, 26b can be sucked up by this injection force to be supplied to the three-fluid nozzle 2, in the case where the viscosity of the first and/or second liquid substance(s) is remarkably high, for example, the first and/or second liquid substance(s) may be guided to the first injection outlet and/or the second injection outlet of the three-fluid nozzle 2 by using a pump or the like so that the superfine particles of the liquid substance(s) can be similarly generated.

Since this inhaler can generate superfine particles of the substance(s) to be sprayed having a uniform particle size, even when it is used for suction tests and the like performed in safety tests of medicines, agrichemicals, and the like, toxicity tests of various chemical substances in working environments, researches on air pollutants and environmental hormones, and the like, accurate test results can be obtained.

If a two-fluid nozzle is used as the fine particle generating nozzle 2 in the embodiment shown in Fig. 1, fine particles of the first liquid substance to be sprayed or the second liquid substance to be sprayed, or a mixture thereof, can be generated by providing the first liquid substance to be sprayed or the second liquid substance to be sprayed, or a mixture thereof, to the fine particle generating nozzle 2, while providing nozzle gas from nozzle gas supplying compressor 34 to the fine particle generating nozzle 2. In this case, the inhaler according to the present invention can spray liquid medicine and the like with a predetermined uniform particle size. In addition, in the inhaler according to the present invention shown in Fig. 1, the vessel wall of the fine particle separating vessel 10 has an auxiliary gas nozzle 19a, and an auxiliary gas supplied from the auxiliary gas compressor 17 is discharged upward from the auxiliary gas nozzle 19a. Therefore, it is possible to perform better the control of the particle size and/or the discharging amount of the fine particles.

The inhaler according to the present invention can sort or separate the fine particles generated from the fine particle generating nozzle in accordance with the particle size thereof in the fine particle separating vessel, and discharge them out. Therefore, it can discharge superfine particle having a very narrow particle size distribution. Thus, it can discharge superfine particles with a uniform particle size.

Accordingly, the present invention can control the maximum particle size of the generated superfine particles to be, for example, less than 1µm. In this case, it is preferable that the maximum particle size of the superfine particles be 500 nm or less, more preferably 300 nm or less, and even more preferably 100 nm or less. If the maximum particle size is less than 1 µm, the superfine particles provided by the present invention do not include any fine particle with a particle size of 1 µm or more. In other words, although fine particles with a mean particle size of less than 1 µm may include a fine particle with a particle size of 1 µm or more, as a part thereof, depending on the particle size distribution, fine particles with a maximum particle size of less than 1 µm do not include such a particle with a relatively large size.

### REFECENCE SIGNS LIST

- 1:: Inhaler
- 2:: Three-fluid nozzle
- 3:: Nozzle body
- 4:: Nozzle cover
- 5:: Gap
- 10:: Fine particle separating vessel
- 14:: Rectifying cone
- 16:: Secondary gas compressor
- 20, 22, 24:: Fine particle sorting plates
- 26:: Storing vessel
- 28:: Discharging section
- 28c:: Suction port
- 28d:: Valve
- 29:: Collecting device
- 32:: Solenoid valve
- 34:: Nozzle gas supplying compressor
- 40:: Power device
- 42:: Control device
- 44a:: Particle size setting section
- 44b:: Particle quantity setting section
- 44c:: Electrical charge setting section
- S1:: Particle size detecting section
- S2:: Particle quantity detecting section

## Claims

1. An inhaler comprising:
at least one fine particle generating nozzle for generating fine particles comprising a first liquid substance to be sprayed and a second liquid substance to be sprayed by atomizing the first liquid substance and the second liquid substance with gas;
at least one separating vessel for separating the fine particles generated by the fine particle generating nozzle; and
at least one discharging section for discharging superfine particles separated from the fine particles in the separating vessel;
a particle size setting means for setting a particle size of the superfine particles to be discharged from the discharging section;
a particle size detecting means for detecting a particle size of the superfine particles to be discharged from the discharging section; and
a first gas pressure control means for controlling the pressure of a gas to be fed to the fine particle generating nozzle such that the particle size of the superfine particles detected by the particle size detecting means is equal to the particle size of the superfine particles set by the particle size setting means,
wherein
the fine particle generating nozzle comprises
a first injection outlet to inject the first liquid substance to be sprayed into the separating vessel,
a second injection outlet to inject the second liquid substance to be sprayed into the separating vessel, and
a gas injection outlet to inject the gas into the separating vessel,
where
the gas injection outlet is arranged around the first injection outlet and the second injection outlet such that the fine particle generating nozzle is capable of generating a fine particle having a core-shell structure in which the core comprises first liquid substance to be sprayed and the shell comprises the second liquid substance to be sprayed.

2. The inhaler according to Claim 1, wherein
the fine particle generating nozzle comprises
a nozzle body having the first injection outlet and the second injection outlet, and
a nozzle cover to form a gap around the nozzle body,
where
the gap connects to the gas injection outlet and a source of the gas, and
the gas injection outlet is capable of injecting the gas around the first injection outlet and the second injection outlet.

3. The inhaler according to Claim 1 or 2, wherein the gas injection outlet is in the shape of a circle.

4. The inhaler according to any one of Claims 1 to 3, wherein the second injection outlet is arranged around the first injection outlet.

5. The inhaler according to any one of Claims 1 to 4, wherein the second injection outlet is in the shape of a circle.

6. The inhaler according to any one of Claims 1 to 5, wherein the first injection outlet is arranged in the second injection outlet.

7. The inhaler according to any one of Claims 1 to 6, wherein the first liquid substance to be sprayed and/or the second liquid substance to be sprayed comprise(s) a liposome.

8. The inhaler according to any one of Claims 1 to 7, further comprising a collecting section for collecting the superfine particle which is not inhaled at the discharging section.

9. The inhaler according to any one of Claims 1 to 8, further comprising:
a particle amount setting means for setting the amount of superfine particles to be discharged from the discharging section;
a particle amount detecting means for detecting the amount of superfine particles to be discharged from the discharging section; and
a liquid substance amount controlling means for controlling the amount of the first liquid substance to be sprayed and/or the amount of the second liquid substance to be sprayed such that the amount of the superfine particles detected by the particle amount detecting means is equal to the amount of superfine particles set by the particle amount setting means.

10. The inhaler according to any one of Claims 1 to 9, further comprising:
a secondary gas feeding means for feeding a secondary gas for elevating the fine particles in the separating vessel; and
a second gas pressure control means for controlling the pressure of the secondary gas to be fed by the secondary gas feeding means such that the particle size of the superfine particles detected by the particle size detecting means is equal to the particle size of the superfine particles set by the particle size setting means.

11. The inhaler according to any one of Claims 1 to 10, wherein the separating vessel comprises:
a flow rectifying member for guiding the fine particles generated by the fine particle generating nozzle to a lower part of the separating vessel; and
at least one fine particle sorting plate for controlling floating up of the fine particles guided to the lower part of the separating vessel by the flow rectifying member and for sorting the fine particles.

12. The inhaler according to Claim 11, wherein a plurality of fine particle sorting plates each of which is provided with a plurality of fine particle passing holes through which fine particles pass are disposed inside the separating vessel, and the size of the fine particle passing holes provided at the fine particle sorting plate positioned at the lower side is larger than the size of the fine particle passing holes provided at the fine particle sorting plate positioned at the upper side.

13. The inhaler according to any one of Claims 1 to 12, further comprising:
an electrical charge supplying means for supplying electrical charges to the superfine particles to be discharged from the discharging section; and
an electrical charge quantity control means for controlling the quantity of electrical charges to be supplied to the superfine particles by the electrical charge supplying means.

14. The inhaler according to any one of Claims 1 to 13, wherein the vessel wall of the separating vessel has a gas introduction means for introducing gas from outside into the separating vessel.

15. An inhaler comprising:
at least one fine particle generating nozzle for generating fine particles comprising a liquid substance to be sprayed by atomizing the liquid substance to be sprayed with gas;
at least one separating vessel for separating the fine particles generated by the fine particle generating nozzle; and
at least one discharging section for discharging superfine particles separated from the fine particles in the separating vessel;
a particle size setting means for setting a particle size of the superfine particles to be discharged from the discharging section;
a particle size detecting means for detecting a particle size of the superfine particles to be discharged from the discharging section; and
a first gas pressure control means for controlling the pressure of a gas to be fed to the fine particle generating nozzle such that the particle size of the superfine particles detected by the particle size detecting means is equal to the particle size of the superfine particles set by the particle size setting means,
wherein
the vessel wall of the separating vessel has a gas introduction means for introducing gas from outside into the separating vessel.
